**Europäisches Patentamt**

**European Patent Office**

**Office européen des brevets**

(11) Veröffentlichungsnummer: **0 188 775**
A1

# (12) EUROPÄISCHE PATENTANMELDUNG

(21) Anmeldenummer: 85116239.6

(22) Anmeldetag: 19.12.85

(51) Int. Cl.⁴: **C 07 C 57/04,** C 07 C 51/48,
C 07 C 51/42

(30) Priorität: 11.01.85 DE 3500678

(71) Anmelder: **Röhm GmbH, Kirschenallee Postfach 4242,
D-6100 Darmstadt 1 (DE)**

(43) Veröffentlichungstag der Anmeldung: **30.07.86
Patentblatt 86/31**

(84) Benannte Vertragsstaaten: **DE FR GB IT NL**

(72) Erfinder: **Gruber, Wilhelm, Dr.,
Peter-Behrens-Strasse 34, D-6100 Darmstadt (DE)**

(54) Verfahren zur Gewinnung wasserfreier Methacrylsäure.

(57) Gewinnung wasserfreier Methacrylsäure aus Gasgemischen wie sie bei der oxydativen Dehydrierung von Isobuttersäure oder der katalytischen Oxydation von Isobutylen bzw. tert.-Butanol oder Methacrolein anfallen, durch Wäsche solcher Gasgemische mit einem inerten, hydrophoben, höher als Methacrylsäure siedendem Lösungsmittel, wie beispielsweise Diphenyl, Diphenyläther, Dibenzofuran und/oder Gemische derselben, und anschliessender Auftrennung des Lösungsmittel-Methacrylsäure-Gemisches.

EP 0 188 775 A1

Verfahren zur Gewinnung wasserfreier Methacrylsäure

Gebiet der Erfindung

Die Erfindung betrifft ein Verfahren zur Gewinnung wasserfreier Methacrylsäure aus gasförmigen Reaktorströmen, wie
sie bei der katalytischen oxydativen Dehydrierung von
Isobuttersäure oder bei der katalytischen Oxydation von
Isobutylen bzw. tert.-Butanol oder Methacrolein in der
Gasphase entstehen.

Stand der Technik

Bei den Synthesen von Methacrylsäure durch katalytische
Oxydation in der Gasphase entsteht sowohl auf dem Weg der
oxydativen Dehydrierung von Isobuttersäure als auch auf dem
Weg der Oxydation von Isobutylen bzw. tert.-Butanol über Isobutylen und
Methacrolein theoretisch pro Mol Methacrylsäure 1 Mol
Wasser. Die bisher bekannten im Temperaturbereich von 300
bis 500°C arbeitenden katalytischen Verfahren laufen weiterhin
nicht sehr selektiv ab, so daß das oben angegebene Wasser-
Methacrylsäure-Molverhältnis von 1 immer deutlich überschritten
wird. Aus Gründen der Selektivitätsverbesserung oder der
verbesserten thermischen Reaktionsführung werden die Oxydationen sehr oft in Gegenwart von zusätzlichem Wasserdampf,
dem sogenannten Moderatorwasser durchgeführt. In der Literatur sind Fälle beschrieben, in denen einem Mol umzusetzendem
Substrat bis zu ca. 20 Mol $H_2O$ zugesetzt werden. Das bedeutet,
daß bei den angegebenen oxydativen Verfahren zur Herstellung

- 2 -

von Methacrylsäure, diese Säure im Gemisch mit mindestens 17,3 Gew.-% Wasser, wie für den theoretischen Fall berechnet, bis zu Wassergehalten von 80 Gew.-% und höher anfällt. Eine einfache Methacrylsäure-Wasser-Trennung durch Destillation scheidet wegen Azeotropbildung aus.

In der US-PS 4 230 887 wird die Gewinnung von wasserfreien Carbonsäuren aus bis ca. 200°C heißen Gasmischungen, die u.a. auch Wasserdampf enthalten, durch Waschen solcher Gasmischungen mit Polyoxyalkylenglykolen oder deren Monoalkyl- und Dialkyläthern nach Methoden der Gasabsorptionstechnik beschrieben. Aus beiden Beispielen, die die Gewinnung von Methacrylsäure beschreiben, die noch 1,8 bzw. 2,0 Gew.-% Wasser enthält, ist ersichtlich, daß man Methacrylsäure mit einem Minimum an Wasser, nämlich noch ca. 2 Gew.-%, erst nach zweistufiger Arbeitsweise erhält. In der ersten Stufe wird auf die Absorptionskolonne, die das Lösungsmittel enthält, noch Wasser aufgegeben, um ein günstiges Hydrophobie-Hydrophilie-Verhältnis des Extraktionsmittels einzustellen. Auf diese Weise erhält man eine Lösung mit relativ hohem Wasseranteil. Dieser Anteil wird durch einen gesonderten Verfahrensschritt in einem Extraktiv-Destillations-Turm weitgehend entfernt, so daß anschließend Methacrylsäure mit oben angegebenen Wassergehalten durch Flash-Destillation aus den Polyoxyalkylenglykol-Lösungen erhalten wird.

Außer diesem Verfahren zur Abtrennung von Methacrylsäure durch Waschen der Gasgemische mit hochsiedendem Lösungsmittel, sind eine Reihe weiterer Verfahren bekannt, bei denen aus den nach Kondensation aus den Reaktionsgemischen erhaltenen

- .3 -

flüssigen Phasen, die Methacrylsäure durch Extraktion mit organischen, hydrophoben Lösungsmitteln vom Wasser getrennt wird. Beispielsweise werden in der J-PS 74 45 020 Hexan, in der J-PS 73 37 012 Cyclohexanon, in der J-PS 77 83 415 Benzol oder in der DE-OS 29 07 602 Xylol bzw. Cumol zusammen mit Methacrylsäuremethylester als Extraktionsmittel genannt.

Für die Abtrennung von Acrylsäure aus Reaktionsgasen, wie sie bei der katalytischen Oxydation von Propylen und/oder Acrolein anfällt, sind Verfahren zur Wäsche der Reaktionsgase mit hochsiedenden organischen Lösungsmitteln beschrieben. So werden als solche nach der DE-OS 16 68 362 Ester von Maleinsäure oder Polyacrylsäuren, die bei der Gasphasenoxydation des Propylens oder Acroleins als Nebenprodukte mitentstehen, verwendet, und nach der DE-OS 21 36 395 werden zur Gewinnung wasserfreier Acrylsäure, hydrophobe Lösungsmittel, wie Kohlenwasserstoffe der Mittelölfraktion oder Diphenyl, Diphenyläther und/oder deren Gemische, eingesetzt.

Zur Abtrennung von Methacrylsäure aus Reaktionsgasen, wie sie bei der Oxydation bzw. der oxydativen Dehydrierung entsprechender Iso-$C_4$-Substrate entsteht, eignen sich die in der DE-OS 16 68 362 angegebenen hochsiedenden organischen Lösungsmittel nicht. Diese sind bei der Durchführung des Verfahrens nicht inert. Sie werden wie in der Patentschrift Seite 3, erster Absatz, letzte Zeile, angegeben, verseift, verunreinigen so  die zu gewinnende Methacrylsäure und erschweren damit deren weitere Reinigung.

Eine Übertragung des Verfahrens der oben angegebenen DE-OS 21 36 396 auf die Abtrennung praktisch wasserfreier Methacrylsäure lag nicht nahe, da die physikalischen und chemischen Eigenschaften, wie z.B. Siedepunkte, Löslichkeiten, Polymerisationsverhalten, von Acrylsäure und Methacrylsäure deutlich verschieden sind. Insbesondere wegen der schon erwähnten Azeotropbildung bei der Destillation des Systems Methacrylsäure-Wasser, die beim System Acrylsäure-Wasser nicht auftritt, war die Gewinnung von praktisch wasserfreier Methacrylsäure in hohen Ausbeuten bei einem analogen Vorgehen nicht zu erwarten.

Aufgabe

Die geschilderten Verfahren zur Gewinnung von praktisch wasserfreier Methacrylsäure weisen erhebliche Nachteile auf: So lehrt die US-PS 4 230 887 das Arbeiten mit Polyoxyalkylenglykolen, d.h. mit aliphatischen Äthern, oder deren Monoalkyl- und Dialkyläthern als Extraktionsmittel, die dabei mit den heißen, noch sauerstoffhaltigen Gasen aus der Oxydationsreaktion in Kontakt gebracht werden. Es ist bekannt, daß aliphatische Äther durch Reaktion mit molekularem Sauerstoff sehr leicht peroxidische Verbindungen bilden, die oft Ursache von heftigen Explosionen sind. Auch bei den Polyalkylenglykolen ist die Oxydationsempfindlichkeit mit zunehmender Temperatur erheblich.
Außer der großen Sauerstoffreaktivität der aliphatischen Polyäther und der damit hohen Gefahr beim Arbeiten mit solchen Systemen, liefert die Arbeitsweise mit den aliphatischen Polyäthern, wie sie weiter oben kurz geschildert wurde, eine bezüglich des Wasseranteils ungenügende Methacrylsäure-Qualität.

Die Verfahren, Methacrylsäure durch Extraktion mit organischen Lösungsmitteln aus wäßrigen Kondensaten zu gewinnen, sind vielstufige Arbeitsverfahren und damit von geringer Wirtschaftlichkeit.

Die Aufgabe, in einem einfachen Verfahren Methacrylsäure und gegebenenfalls nicht umgesetzte Ausgangsverbindungen in wirtschaftlicher Weise und ohne Bildung unerwünschter und gefährlicher Verbindungen von Wasser und nicht kondensierbaren Gasgemischkomponenten aus heißen Reaktionsgemischen abzutrennen, ist bisher noch nicht befriedigend gelöst worden.

Lösung

Es wurde nun ein Verfahren zur Lösung dieser Aufgabe gefunden, das darin besteht, daß man methacrylsäurehaltige Gasgemische, wie sie bei der oxydativen Dehydrierung von Isobuttersäure oder bei der Isobutylen- bzw. tert.-Butanol- und der Methacroleinoxidation entstehen, einer Wäsche mit einem hochsiedenden organischen, hydrophoben und inerten Lösungsmittel unterwirft und aus der erhaltenen organischen Lösung dann die Methacrylsäure sowie gegebenenfalls unumgesetzte Vorprodukte, wasserfrei, nach an sich bekannten Verfahren abtrennt. Hochsiedende, hydrophobe und inerte organische Lösungsmittel, die für das Verfahren geeignet sind, sind aromatische Verbindungen mit mehreren, gegebenenfalls über Sauerstoffatome verbundenen Phenylkernen, wie z.B. Diphenyl, Diphenyläther oder Dibenzofuran und bevorzugt Gemische dieser Verbindungen. Besonders bevorzugt wird ein Gemisch aus Diphenyl und Diphenyläther wie es unter der Bezeichnung "Diphyl" als organischer Wärmeüberträger im Handel erhältlich ist und ein eutektisches Gemisch von 26,5 Gew.-% Diphenyl und 73,5 Gew.-% Diphenyläther mit einem Erstarrungspunkt von 12,3°C und einem Siedepunkt von ca. 257°C darstellt.

Die Gaswäsche kann so durchgeführt werden, daß man das zunächst noch 150 bis 350°C heiße Gasgemisch durch die praktisch ruhende Absorptionsflüssigkeit leitet oder zweckmäßigerweise das heiße Gasgemisch in einer Absorberkolonne mit dem hydrophoben inerten Lösungsmittel im Gegenstrom wäscht. Bei dem erfindungsgemäßen Verfahren müssen Druck und Temperatur bei der Absorption in Abhängigkeit vom Wasseranteil der methacrylsäurehaltigen Gasgemische so eingestellt werden, daß keine wäßrige Phase anfällt. Als günstiger Temperaturbereich in der Absorptionsflüssigkeit hat sich beim Arbeiten bei Normaldruck ein solcher von 40 bis 120°C erwiesen. Der Gehalt der Absorberflüssigkeit an Methacrylsäure und noch gegebenenfalls vorhandenem nicht umgesetztem Ausgangsprodukt wie z.B. Isobuttersäure, liegt je nach Arbeitsbedingungen zwischen 5 bis 30 Gew.-%, und der Anteil des Wassers bezogen auf die absorbierte Menge an Methacrylsäure bzw. Methacrylsäure und Ausgangsprodukt unter 1 %, insbesondere zwischen 0,3 und 0,7 Gew.-%. Bei der Oxydehydrierung von Isobuttersäure werden u.a. auch Aceton und Essigsäure gebildet, die bei der Aufarbeitung nach dem erfindungsgemäßen Verfahren im wesentlichen mit dem Wasser und den übrigen Gasgemischanteilen über Kopf abgehen und deren Anteile im Absorberablauf bezogen auf die abgetrennte Methacrylsäure bzw. Methacrylsäure-Isobuttersäure ebenfalls deutlich unter 1 Gew.-% liegen. Die Aufarbeitung von methacrylsäurehaltigen Gasgemischen aus der Isobutylen- bzw. tert.-Butanol- und Methacroleinoxidation liefert Lösungen, die außer den oben schon angegebenen Nebenprodukten Aceton und Essigsäure noch die entsprechenden Oxidationsausgangsverbindungen in geringer Menge enthalten können.

Die bei dem erfindungsgemäßen Verfahren behandelten Gasgemische kann man allgemein so beschreiben, daß sie 50 bis 90 Vol.-% Stickstoff, 0,5 bis 10 Vol.-% Sauerstoff, 0,1 bis 5 Vol.-% CO, 0,1 bis 5 Vol.-% $CO_2$, 1 bis 50 Vol.-% Wasserdampf, 0,5 bis 15 Vol.-% Methacrylsäure, 0,1 bis 1 Vol.-% Essigsäure, 0,1 bis 2 Vol.-% Aceton, 0,01 bis 2 Vol.-% Propylen und gegebenenfalls noch Ausgangsverbindungen wie Isobutylen, tert.-Butanol, Methacrolein oder Isobuttersäure in Mengen bis 5 Vol.-% enthalten.

Der Absorberablauf wird nach bekannten Destillationstechniken zur Gewinnung der reinen Methacrylsäure oder des Methacrylsäure-Isobuttersäuregemisches aufgearbeitet. Dazu kann dieser zur weitgehendst vollständigen Entfernung des noch vorhandenen Wassers und der anderen niedrigsiedenden Verbindungen zweckmäßigerweise zuerst in einer Desorberkolonne mit einem Stripgas, z.B. Luft, im Gegenstrom behandelt werden, wobei die Desorptionstemperaturen etwa 30°C höher als die Absorptionstemperaturen liegen. Nach der Desorberoperation wird die zurückbleibende Lösung einer Destillation unterworfen, und die Methacrylsäure bzw. ein Methacrylsäure-Isobuttersäure-Gemisch, das in einer weiteren Destillation in Methacrylsäure und Isobuttersäure getrennt wird, gewonnen.

- .8 -

Zur Verhinderung einer Polymerisation von Methacrylsäure zu hochsiedenden Substanzen werden den Lösungsmitteln bzw. den Lösungen noch an sich übliche, bekannte Stabilisatoren wie z.B. Hydrochinon oder Hydrochinonmonomethyläther zugesetzt, die mit dem noch im Reaktionsgas vorhandenen oder dem Stripgas zugesetzten Sauerstoff wirksame Polymerisationsinhibitorsysteme bilden.

Durch ständige Auskreisung eines Teilstromes des Lösungsmittels und dessen Reinigung durch Destillation mit anschließender Rückführung in die Trennanlage, wird eine Anreicherung hochsiedender und praktisch nicht verdampfbarer Verbindungen im Lösungsmittel vermieden.

## Beispiel 1

In den unteren Teil einer Absorptionskolonne werden 1500 Nl 250 bis 270°C heißes Reaktionsgas, das neben Inertgasen wie $N_2$, $CO_2$, CO, 3,9 Vol.-% Methacrylsäure, 7,23 Vol.-% Wasserdampf[*], 0,41 Vol.-% Aceton, 0,03 Vol.-% Essigsäure, und 4,1 Vol.-% $O_2$ enthält, innerhalb von 3 Stunden geleitet. Am Kopf der Kolonne wird als hochsiedendes, inertes und hydrophobes Extraktionsmittel ein auf 40°C gehaltenes Gemisch von 73,5 Gew.-% Diphenyläther und 26,5 Gew.-% Diphenyl, als "Diphyl" bekannt und 25 ppm Hydrochinon enthaltend, mit einer Zugabegeschwindigkeit von 500 g/h eindosiert.

Am unteren Kolonnenausgang wird eine ca. 90°C heiße Diphyllösung, enthaltend 13,0 Gew.-% Methacrylsäure, 0,08 Gew.-% Wasser, Spuren von Aceton und Essigsäure, entnommen. Die Vakuumdestillation dieser Lösung mit Vigreuxkolonne erbringt eine Methacrylsäure in einer Reinheit von 99,7 % und in einer Menge von 96,4 % der in der Diphyllösung erhaltenen Methacrylsäure. Das als Sumpf zurückbleibende Diphyl enthält noch 3,6 % der im Diphyl eingebrachten Methacrylsäuremenge, die mit dem Lösungsmittel in die Absorptionskolonne zurückgeführt wird.

## Beispiel 2

675 Nl Reaktionsgas, enthaltend 2,51 Vol.-% Methacrylsäure, 0,91 Vol.-% Isobuttersäure, 4,07 Vol.-% Wasserdampf, 0,48 Vol.-% Aceton, 0,04 Vol.-% Essigsäure, 3,68 Vol.-% Sauerstoff, Rest Inertgase wie $N_2$, $CO_2$, CO, Propylen, wird, mit einer Temperatur von 250 bis 300°C ankommend, innerhalb von 2,8

[*] 0,35 Vol.-% Propylen

- .10 -

Stunden aufsteigend durch 500 g auf 80°C temperiertes, 25 ppm Hydrochinon enthaltendes "Diphyl" geleitet. Die Analyse zeigt, daß die erhaltene Diphyllösung 11,2 Gew.-% Methacrylsäure, 3,7 Gew.-% Isobuttersäure, 0,1 Gew.-% Essigsäure, 0,07 Gew.-% Aceton und 0,04 Gew.-% Wasser enthält.

Beispiel 3

1543 Nl ca. 280°C heißes Reaktionsgas, enthaltend 2,11 Vol.-% Methacrylsäure, 1,63 Vol.-% Isobuttersäure, 2,94 Vol.-% Wasserdampf, 0,39 Vol.-% Aceton, 0,02 Vol.-% Essigsäure, 4,04 Vol.-% Sauerstoff, Rest Inertgase wie $N_2$, $CO_2$, CO, Propylen, wird innerhalb von 3 Stunden aufsteigend durch 500 g auf 96°C gehaltenem "Diphyl" mit 25 ppm Hydrochinonzusatz geleitet. Die Analyse zeigt, daß die erhaltene Diphyllösung 16,3 Gew.-% Methacrylsäure, 12 Gew.-% Isobuttersäure, 0,02 Gew.-% Essigsäure, 0,06 Gew.-% Aceton und 0,2 Gew.-% Wasser enthält.

Die nach den Beispielen 2 und 3 erhaltenen Diphyllösungen werden durch Destillation in einer Kolonne mit relativ hoher Bodenzahl in Isobuttersäure, die in die Oxydehyrierung zurückgeführt wird, in das Prozeßprodukt Methacrylsäure und in Diphyl als Sumpfprodukt, das in den Trennprozeß zurückgeht, aufgetrennt.

Beispiel 4

Nach Beispiel 1 wird anstelle des dort eingesetzten Diphenyläther-Diphenyl-Gemisches, ein Extraktionsmittel-Gemisch aus 60 Gew.-% Diphenyläther und 40 Gew.-% Dibenzofuran, das noch 30 ppm Hydrochinon enthält, mit 1 000 Nl 2 Stunden lang mit einem wie in Beispiel 1 angegebenen Reaktionsgas behandelt. Aus der erhaltenen Lösung mit 13,1 Gew.-% Methacrylsäure, 0,1 Gew.-% in Wasser, 0,05 Gew.-% Essigsäure und 0,05 Gew.-% Aceton, werden durch Vakuumdestillation 96 % der im Extraktionsmedium enthaltenen Methacrylsäure in einer Reinheit von 99,6 % erhalten.

Beispiel 5

Beispiel 4 wird mit reinem Diphenyläther, enthaltend 50 ppm Hydrochinonmonomethyläther, als Extraktionsmittel wiederholt. Aus dem bei der Extraktion anfallenden Gemisch mit 12,9 Gew.-% Methacrylsäure, 0,1 Gew.-% Wasser, 0,1 Gew.-% Essigsäure und kleiner 0,1 Gew.-% Aceton, wird praktisch reine Methacrylsäure in 98 % der im Extraktionsgemisch enthaltenen Menge erhalten.

Beispiel 6

1 000 Nl Reaktionsgas der Zusammensetzung nach Beispiel 1, werden innerhalb von 2 Stunden aufsteigend durch 1 000 g auf 90°C temperiertes flüssiges Diphenyl, enthaltend 50 ppm Hydrochinonmonomethyläther geleitet. Die gaschromatographische Analyse der erhaltenen Diphenyllösung zeigt einen Gehalt von 11,5 Gew.-% Methacrylsäure, 0,1 Gew.-% Essigsäure und Spuren von Wasser und Aceton an. Durch Vakuumdestillation wird praktisch reine Methacrylsäure aus der Lösung in etwa 97 % der enthaltenen Menge gewonnen.

- .12 -

Beispiel 7

450 Nl ca. 250°C heißes Reaktionsgas aus einer katalytischen Oxidation von Methacrolein in der Gasphase, enthaltend 2,6 Vol.-% Methacrylsäure, 25,3 Vol.-% Wasserdampf, 0,2 Vol.-% Essigsäure, 0,1 Vol.-% Aceton, 4,2 Vol.-% Sauerstoff, 0,9 Vol.-% Methacrolein, Rest die Inertgase, insbesondere $N_2$ und weiter noch $CO_2$ und CO, werden innerhalb von 2 Stunden aufsteigend durch 500 ml einer auf 80°C temperierten Mischung aus 80 Gew.-% "Diphyl" und 20 Gew.-% Dibenzofuran, die noch 50 ppm Hydrochinon enthält, geleitet. Die erhaltene Extraktionslösung enthält nach Analyse 8 Gew.-% Methacrylsäure, 0,1 Gew.-% Essigsäure, Spuren von Aceton, 0,1 Gew.-% Methacrolein und 0,06 Gew.-% Wasser. Aus dieser Lösung wird die Methacrylsäure nach Abtrennung der übrigen Bestandteile durch Vakuumdestillation in 99,8 %iger Reinheit und in einer Menge von 97 % der in der Lösung vorhandenen Menge erhalten.

Beispiel 8

520 Nl 240°C heißes Reaktionsgas aus einer katalytischen Isobutylen-Oxidation, enthaltend 2,9 Vol.-% Methacrylsäure, 20,3 Vol.-% Wasserdampf, 0,4 Vol.-% Essigsäure, 0,1 Vol.-% Isobutylen, 6,2 Vol.-% Sauerstoff, sowie noch $N_2$, $CO_2$ und CO werden innerhalb von 2 Stunden aufsteigend durch 500 ml auf 80°C temperiertes "Diphyl" geleitet. Die erhaltene Extraktionslösung enthält 9,7 Gew.-% Methacrylsäure, 0,15 Gew.-% Essigsäure und 0,1 Gew.-% Wasser, aus der die Methacrylsäure in praktisch reiner Form durch Destillation gewonnen wird.

Beispiel 9

Mit tert.-Butanol anstelle von Isobutylen als Ausgangsverbindung wird bei gleichem Vorgehen wie in Beispiel 8 beschrieben, praktisch das gleiche Ergebnis erhalten.

Verfahren zur Gewinnung wasserfreier Methacrylsäure

Patentansprüche

1.  Verfahren zur Gewinnung wasserfreier Methacrylsäure aus bei der katalytischen oxydativen Dehydrierung von Isobuttersäure oder der katalytischen Oxydation von Isobutylen bzw. tert.-Butanol oder Methacrolein in der Gasphase anfallenden Gasgemischen durch Wäsche, insbesondere Gegenstromwäsche, der Reaktionsgase mit einem organischen Lösungsmittel, das höher als alle abzutrennenden Produkte siedet,

    dadurch gekennzeichnet,

    daß man die Wäsche mit einem inerten, hydrophoben Lösungsmittel durchführt und das dabei erhaltene Lösungsmittel-Methacrylsäure-Gemisch in bekannter Weise weiter aufarbeitet.

2.  Verfahren nach Anspruch 1, dadurch gekennzeichnet, daß man als inerte, hydrophobe Lösungsmittel aromatische Verbindungen mit mehreren, gegebenenfalls über Sauerstoffatome verbundenen, Phenylkernen verwendet.

3.  Verfahren nach den Ansprüchen 1 und 2, dadurch gekennzeichnet, daß man als inerte, hydrophobe Lösungsmittel Diphenyl, Diphenyläther, Dibenzofuran und/oder deren Gemische verwendet.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE-A-1 768 280 (ESCAMBIA) <br> * Ansprüche 1-3 * <br><br> --- | 1 | C 07 C 57/04 <br> C 07 C 51/48 <br> C 07 C 51/42 |
| D,Y | DE-A-2 136 396 (BASF) <br> * Ansprüche 1,2 * <br><br> --- | 1-3 | |
| A | GB-A- 354 198 (DREYFUS) <br> * Ansprüche 1,2,4,5,7 * <br><br> ----- | 1-3 | |
| | | | **RECHERCHIERTE SACHGEBIETE (Int. Cl.4)** <br><br> C 07 C 57/00 <br> C 07 C 51/00 |

Der vorliegende Recherchenbericht wurde für alle Patentansprüche erstellt.

| Recherchenort <br> DEN HAAG | Abschlußdatum der Recherche <br> 18-04-1986 | Prüfer <br> KLAG M.J. |
|---|---|---|